# EUROPÄISCHE PATENTSCHRIFT

(11) **EP 1 980 839 B1**
(45) Veröffentlichungstag und Bekanntmachung des Hinweises auf die Patenterteilung: **05.06.2013**
(21) Anmeldenummer: 08153446.3
(22) Anmeldetag: 27.03.2008
(51) Int. Cl.: G01N 21/25, G01N 21/89, B07C 5/342, G01B 11/25, G01N 33/12, G01N 33/02

(54) **Kontrollvorrichtung für Lebensmittel und deren Verwendung**
Inspection device for food and its use
Dispositif de contrôle pour produits alimentaires et son utilisation

(30) Priorität: 10.04.2007 US 733277
(43) Veröffentlichungstag der Anmeldung: 15.10.2008
(73) Patentinhaber: Bizerba GmbH & Co. KG, 72336 Balingen (DE)
(72) Erfinder: Mulder, Steven C., Mechanicsville, VA 23116 (US); Gray, Calvin G., Richmond, VA 23226 (US); Scardino, Paul G., Layton, UT 84040 (US)
(74) Vertreter: Hoeger, Stellrecht & Partner Patentanwälte

(56) Entgegenhaltungen:
- WO-A-00/62983
- WO-A-95/21375
- DE-A1- 2 728 913
- DE-A1- 4 331 772

## Beschreibung

Die vorliegende Erfindung betrifft eine Kontrollvorrichtung für Lebensmittel entweder zur Identifizierung oder auch zur Sortierung von insbesondere in einer Schale verpackten Lebensmittelprodukten.

Die Erfindung betrifft weiterhin die Verwendung einer Kontrollvorrichtung zur Identifizierung und Sortierung von Lebensmittelprodukten.

In der Praxis werden Lebensmittel automatisiert verpackt, verwogen und transportiert.

Die WO 02/27281 A1 beschreibt ein Wägesystem für das Wiegen von Artikeln, die auf einem Förderband transportiert werden, das mindestens eine erste und eine zweite Fördereinheit umfasst, das Wägesystem, das eine erste und eine zweite Wägezelle, die entsprechend die erste und zweite Fördereinheit stützt, umfasst und so angepasst ist, dass es Daten bezüglich des Gewichts eines oder mehrerer Artikel liefert, die von besagten Fördereinheiten gestützt werden, sowie ein Kontrollsystem, das die Daten von zumindest der Stelle einer oder mehrerer Ränder des Artikels auf dem Förderband liefert, eine Prozessoreinheit zur Verarbeitung der von den Erkennungssystemen und Wägezellen gelieferten Daten und zur Festlegung von zumindest dem Gewicht des/r Artikel/s und zur Speicherung des ermittelten Gewichts.

Die WO 98/42196 A1 beschreibt eine Vorrichtung zum Entfernen von Rippenteilen von der Bauchseite eines Tieres, einschließlich der Transportmittel, Mittel für die Bildabtastung, für die Generierung von Daten zum Oberflächenprofil, die die Oberfläche der Seite beschreiben, für die Generierung von Daten zur Rippendicke, einem Prozessor, einem Roboter, ausgerüstet mit einer Schneidevorrichtung und einer Steuerung, die verwendet wird, um die relative Bewegung zwischen den Schneidevorrichtungen und der Seite zu produzieren, entsprechend programmierter Schneidedaten.

Die US 6,133,948 A beschreibt ein automatisches Erkennungssystem, einschließlich einer ersten Klassifikationsstation, die zwei Laserquellen und Videobildtechniken verwendet, um Objekte zu erkennen und zwischen ähnlichen Objekten zu unterscheiden, z. B. Schranktüren aus Holz und Schubladenfront.

Die WO 95/21375 A1 offenbart ein System für die Onlinebestimmung von Qualitätscharakteristika von Fleischstücken für die Qualitätskontrolle oder für einen Sortiervorgang. Ein Förderband fördert die Fleischstücke, wobei eine Beleuchtungsanordnung über dem Förderband zur Beleuchtung der Fleischstücke angeordnet ist. Eine Farbkamera ist positioniert, um drei Rahmen der Fleischstücke in dem roten, grünen und blauen Spektralbereich aufzunehmen.

Die DE 27 28 913 A1 offenbart ein Verfahren zum Klassifizieren von Fleisch, bei dem an definierten Stellen einer Schlachtkörperhälfte eine mechanische und/oder elektro-optische Messung mehrerer für die Bestimmung der Handelsklasse maßgebender Parameter durchgeführt wird, die Messwerte in elektrische Signale umgewandelt und in einem Steuer- und Speichergerät in Relation zur vorgegebenen bestimmten Handelsklassen zugeordneten Werten gesetzt werden und ein Klassifizierungsmerkmal auf die Schlachtkörperhälfte aufgebracht wird.

Aus der DE 4331772 A1 ist eine Vorrichtung zur Farberkennung von in den Näpfen von Blisterfolien liegenden Objekten mit einer ein breites Spektrum sichtbaren Lichts erzeugenden Auflichtbeleuchtungseinheit und einer Farbkamera, die das von Objekten reflektierte Licht der Auflichtbeleuchtungseinheit erfasst und der Weiterverarbeitung zuführt, bekannt.

Die WO 00/62983 A1 offenbart eine Vorrichtung zur Repräsentation des Oberflächenprofils eines Produkts zur Verwendung bei der nachfolgenden Bearbeitung des Produkts.

Es ist Aufgabe der vorliegenden Erfindung, eine Kontrollvorrichtung für Lebensmittel zu schaffen, so dass Lebensmittelprodukte automatisch erkannt und sortiert werden können. Insbesondere soll Zudem eine automatische Gütebestimmung von Lebensmitteln möglich sein.

Gelöst wird diese Aufgabe mit einer Kontrollvorrichtung mit den Merkmalen von Anspruch 1.

Indem von einer Kamera aufgenommene Bilder von Lebensmittelprodukten, vorzugsweise von in Schalen verpackten Lebensmitteln, mittels einer Evaluierungsvorrichtung hinsichtlich Farbe und/oder Helligkeit analysiert werden, können die Lebensmittelprodukte, insbesondere die in den Produkten enthaltenen Lebensmittel wie Wurstwaren, Fleischwaren und/oder Käse und/oder deren Verpackungen identifiziert werden. Aufgrund der Identifikation können die Lebensmittelprodukte automatisch sortiert oder vorzugsweise automatisch etikettiert werden.

Die Kontrollvorrichtung umfasst dabei unter anderem einen Positionierungsbereich und insbesondere Schalenpositionierungsbereich, ein Beleuchtungsgerät zur Beleuchtung des Lebensmittels mit weißem Licht und mindestens eine Kamera für die Aufnahme von Bildern des beleuchteten Lebensmittels. Mittels der Evaluierungsvorrichtung bzw. des Evaluierungsgeräts werden die von der Kamera aufgenommenen Bilder verarbeitet. Die Evaluierungsvorrichtung führt dabei insbesondere die Farbanalyse der aufgenommenen Bilder und/oder eine Helligkeitserkennung durch.

Bei der vorliegenden Erfindung wird das Lebensmittelprodukt und/oder die Schale mit weißem Licht beleuchtet. So können der (Schalen-)Positionierungsbereich und damit das Lebensmittelprodukt und die Schale mit weißem Licht in einem breiten Spektrum beleuchtet werden. Die Farbe der aufgenommenen Bilder kann auf einfache Weise analysiert werden. Es ist leicht, ein Bild mit hoher Auflösung zu erzielen und damit das Lebensmittelprodukt richtig zu bestimmen. Zudem kann aus einem Kamerabild eine Helligkeitsinformation oder eine Intensitätsinformation einer bestimmten Farbe leicht entnommen werden.

Das weiße Licht setzt sich aus rotem, grünen und blauem Licht zusammen. Bei der Farbanalyse werden verschiedene Farben aufgelöst und diese zur Festlegung von Eigenschaften des Lebensmittelprodukts und seiner Verpackung verwendet.

In einer Ausführung kann vorgesehen sein, dass mit einer Schwarzweiß-Kamera gearbeitet wird, um Herstellungskosten zu reduzieren. In diesem Fall entsprechen die unterschiedlichen Farben unterschiedlichen Helligkeitswerten, die dann von der Evaluierungsvorrichtung ausgewertet werden. Zudem können Farbfilter verwendet werden, um den Kontrast der Helligkeitswerte zu steigern.

Erfindungsgemäß werden die Konturen des Lebensmittelprodukts von denen der Schale auf einfache Art unterschieden, indem die Evaluierungsvorrichtung die Bilder mittels eines Kantenfilters oder eines Konturfilters verarbeitet. Erfindungsgemäß werden zudem über eine Konturerkennung und einen Vergleich mit Solldaten einer fehlerfreien Verpackung auf einfache Weise Einschlüsse in einer Versiegelung erkannt. Solche Einschlüsse können beispielsweise Fremdkörper oder fehlerhaft verpackte Lebensmittel sein.

Außerdem ist es möglich, auf einfache Art eine Laserlinie über dem Lebensmittelprodukt und der Schale zu analysieren, wenn die entsprechende Lichtlinie eine Farbe, wie z. B. Grün, Rot oder Blau hat.

Normalerweise ist das weiße Licht eine Überlagerung aus rotem, grünem und blauem Licht. (Andere Farben können jedoch mit eingeschlossen werden.) Die Farbanalyse kann im Hinblick auf Rot, Grün und Blau durchgeführt werden. So ist es z. B. möglich, Eigenschaften von Fleischprodukten zu unterscheiden.

In einer Ausführung umfasst die Beleuchtungsvorrichtung eine Quelle für weißes Licht. Die Beleuchtungsvorrichtung umfasst beispielsweise eine oder mehrere Dioden, die Breitbandlicht abgeben.

Die Beleuchtungsvorrichtung kann auch Quellen für rotes, grünes und blaues Licht einschließen. In diesem Fall wird das weiße Licht als Überlagerung aus dem Licht verschiedener Quellen generiert.

Es ist von Vorteil, wenn ein Reflexionsbereich zur Verfügung steht, von dem aus das weiße Licht in Richtung des Schalenpositionierungsbereichs gerichtet wird. Der Reflexionsbereich ist eine Art Quelle für weißes Licht. Er kann beispielsweise durch Plastikplatten gebildet werden, die das Licht nach unten auf den (Schalen-)Positionierungsbereich reflektieren. So kann die Spiegelung auf einen Deckel minimal gehalten werden.

Es ist eine Ausführung vorgesehen, bei der die Lichtquellen das Lebensmittelprodukt mit polarisiertem Licht beleuchten, um Lichtspiegelungen zu minimieren. Es wird ein Polarisationsfilter verwendet, der zwischen einer Lichtquelle und dem Lebensmittelprodukt angeordnet ist und nur polarisiertes Licht passieren lässt. Es kann jedoch auch vorgesehen sein, den Polarisationsfilter vor einer Kamera anzuordnen, um etwaige Spiegelungen auszublenden.

Vor allem das Licht der Quellen für rotes, grünes und blaues Licht wird in Richtung des Reflexionsbereichs gerichtet und in Richtung eines Reflexionsbereichs reflektiert.

Es bietet sich an, wenigstens eine Leuchtdiode als Lichtquelle zu haben. Leuchtdioden verbrauchen vergleichsweise wenig Energie und können raumsparend angeordnet werden. Außerdem produzieren Leuchtdioden vergleichsweise wenig Wärme. Es können eine oder mehrere weiße Breitband-Leuchtdioden verwendet werden oder eine oder mehrere Leuchtdioden für verschiedene Farben, wie Rot, Grün und Blau.

Für den Schalenpositionierungsbereich bietet sich ein umgebendes Gehäuse an. So können das Lebensmittelprodukt (und eine Schale) gezielt beleuchtet und die Beeinträchtigung durch störendes Licht minimal gehalten werden.

Sinnvollerweise verfügt das Gehäuse über Öffnungen, durch die die Schalen geführt werden können. So können die Schalen z. B. durch eine entsprechende Kontroll-Station über ein Förderband geführt werden.

Besonders sinnvoll ist es, wenn innerhalb der Sichtweite der Kamera(s) Farbreferenzfelder angeordnet sind. Diese Farbreferenzfelder, die z. B. durch entsprechende rote, grüne und blaue Farbflächen gebildet werden, dienen als Referenzfarben. Die Kamera(s) können die Leuchtdichte und die Farbbalance überwachen. Die Einstellungen der Kamera(s) können ggf. automatisch angepasst werden. Auf diese Weise kann eine hohe Verlässlichkeit des Systems erzielt werden.

Es ist von Vorteil, wenn mindestens ein Farbreferenzfeld für die Farbe Weiß verfügbar ist. So kann eine Kamera am entsprechenden weißen Ziel dieses Referenzfelds bzw. dieser Referenzfelder ausgerichtet werden und z. B. ein Vergleich zwischen Weiß und Rot in einer roten Ebene, Weiß und Grün in einer grünen Ebene usw. versucht werden.

Die Farbreferenzfelder sind idealerweise am oder nahe des Schalenpositionierungsbereichs angeordnet. Die Referenzfelder befinden sich damit im Sichtfeld der Kamera.

Sinnvollerweise wird/werden das/die Evaluierungsgerät/e so angepasst, dass ein Bild im Hinblick auf die Positionen der Farben Rot, Grün und Blau analysiert werden kann. So ist es möglich, verschiedene Farbbereiche in einem Bild zu unterscheiden und das Lebensmittelprodukt auf verschiedene Bereiche hin zu analysieren. Zum Beispiel können bei einem Fleischprodukt Knochenbereiche, fette Bereiche und magere Bereiche unterschieden werden. Über die Erkennung der unterschiedlichen Bereiche kann die Produkterkennung verbessert werden und z.B. automatisch zwischen verschiedenen Lebensmitteln unterschieden werden.

Erfindungsgemäß ist das Evaluierungsgerät zur Identifizierung der Kontur oder der Form des Lebensmittelprodukts in der Schale und zur Unterscheidung des Lebensmittelprodukts von der Schale angepasst. So kann die Lebensmittelproduktanalyse auf das Bild des Lebensmittelprodukts beschränkt werden. Informationen über die Kontur oder die Form eines Lebensmittelprodukts können zur Bestimmung des Lebensmittelprodukts verwendet werden. Auch Informationen zur Form einer Schale könnten nützliche Informationen sein. Zudem kann die Bildanalyse eine Größenerkennung des Lebensmittels und/oder der Schale ergeben, um weitere Informationen zur Identifizierung oder Sortierung des Lebensmittelprodukts zu erhalten.

Falls das System ein Lasergerät umfasst, das mindestens eine lasergenerierte Lichtlinie über eine Schale produziert, können weitere Evaluierungsschritte (Evaluierungsmodule) durchgeführt werden. Über die Laserlinie ist es beispielsweise möglich, die Höhe einer Schale und die Höhe der Rundung eines Foliendeckels zu ermitteln und es ist möglich zu überprüfen, ob es unter dem Deckel Einschlüsse gibt, insbesondere unter einem versiegelten Bereich des Deckels. Außerdem ist es durch Analyse der Form der lasergenerierten Lichtlinie über dem Lebensmittelprodukt in einem entsprechenden Bild möglich, das Lebensmittelprodukt selbst zu bestimmen.

Das Lasergerät sollte Licht in einem Winkel (schräg) zur Ebene, senkrecht zum Schalenpositionierungsbereich, abgeben. So kann anhand des Versatzes zwischen einem Laserbereich auf einer Erweiterung der Schale (Schalenrand) und einem lasergenerierten Lichtbereich auf dem Schalenpositionierungsbereich die Höhe der Schale ermittelt werden. Mit einer lasergenerierten Lichtlinie auf einem Foliendeckel ist es außerdem möglich, die Höhe der Rundung des Foliendeckels zu ermitteln.

Für die Prüfung von Fleischprodukten ist es besonders vorteilhaft, wenn das Lasergerät grünes Licht generiert. Das Evaluierungsgerät kann auf diese Weise leicht den Laserlichtbereich in einem Bild ermitteln. Für andere Arten von Lebensmittelprodukten sind möglicherweise andere Laserlichtfarben von Vorteil.

Das Evaluierungsgerät ist vor allem darauf ausgerichtet, eine lasergenerierte Lichtlinie bezüglich der Segmentierung zu analysieren. Die Segmentierung einer Laserlichtlinie über einem Lebensmittelprodukt kann wertvolle Informationen über das Lebensmittelprodukt liefern.

Es können ein Segment, die Länge der Segmente, die Länge der Segmentverteilung, der Winkel der Segmente, die Winkelverteilung der Segmente oder der Versatz der Segmente analysiert werden. Auch die Textur des Lebensmittelprodukts um eine lasergenerierte Lichtlinie kann analysiert werden.

Außerdem können die Form des Lebensmittelprodukts, die Form der Bereiche des Lebensmittelprodukts, die Verteilung der Form der Bereiche des Lebensmittelprodukts, die Größe der Bereiche des Lebensmittelprodukts oder die Verteilung der Größe der Bereiche des Lebensmittelprodukts mit dem Evaluierungsgerät analysiert werden.

Mit dem Kontrollsystem für Lebensmittel in Anlehnung an die vorliegende Erfindung können auch Schalen analysiert werden, die mit einem durchsichtigen Deckel, z. B. einem Foliendeckel, versiegelt sind.

Das Evaluierungsgerät evaluiert z. B. eine lasergenerierte Lichtlinie, die durch Lichtreflexion auf dem Deckel zustande kommt. Mit dieser Lichtlinie, verglichen mit einem lasergenerierten Lichtbereich auf einer Erweiterung der Schale, kann die Höhe der Rundung des Deckels ermittelt werden.

In Anlehnung an die vorliegende Erfindung ist es möglich, über das Evaluierungsgerät die Höhe der Schale durch einen Vergleich des Versatzes des Lichts, das vom Schalenpositionierungsbereich oder einem Bereich in der Nähe dieses Bereichs reflektiert wird und der lasergenerierten Lichtlinie auf einer Erweiterung der Schale, zu ermitteln.

Erfindungsgemäß wird ein versiegelter Bereich der Schale im Hinblick auf Einschlüsse, vorzugsweise Fremdkörper oder fehlverpackte Lebensmittel,analysiert. Der versiegelte Bereich der Schale hat dieselbe Farbe wie die Schale, d. h. sollte es in diesem Bereich eine Farbveränderung geben, müssen Einschlüsse vorhanden sein.

In Anlehnung an die vorliegende Erfindung ist es von Vorteil, wenn das System eine erste und mindestens eine zweite Kamera zum Scannen eines lasergenerierten Lichtbereichs umfasst. Mit einer zweiten Kamera kann beispielsweise die Versiegelung im Hinblick auf Einschlüsse geprüft werden. Mit einer zweiten Kamera ist es z. B. möglich, kontinuierlich den Versiegelungsbereich zu prüfen.

Das System kann mit einem vorzugsweise motorisch angetriebenen Förderband zum Transport der Schalen ausgestattet sein. Auf diese Weise kann eine hohe Prüfrate erzielt werden.

Idealerweise umfasst die Evaluierungsvorrichtung einen Datenspeicher für gespeicherte Produkterkennungsdaten und/oder -sortierungsdaten. Auf diese Weise kann die Evaluierungsvorrichtung numerische Werte für durchgeführte Analysemodule berechnen und diese mit den vorliegenden Daten zu bekannten Lebensmittelprodukten vergleichen. So kann die Evaluierungsvorrichtung unterschiedliche Lebensmittelprodukte erkennen und die Daten dann an eine Etikettierstation oder eine Sortierstation weitergeben, so dass die Lebensmittelprodukte dann z. B. automatisch etikettiert und/oder sortiert werden können.

Das Evaluierungsgerät kann mit einem "Lernbereich" ausgestattet werden, so dass neue Produkte eingeführt werden können. Ein neues Produkt kann durch das System geschickt und die entsprechenden Daten können abgerufen werden. Sollte das Lebensmittelprodukt mit den gespeicherten Produkten nicht übereinstimmen, können die entsprechenden Daten gespeichert werden.

Ein Verfahren zur Identifizierung und Sortierung von in einer Schale verpackten Lebensmittelprodukten besteht aus der Evaluierung der Form des Lebensmittelprodukts in der Schale und der Analyse des Lebensmittelprodukts hinsichtlich der verschiedenen Bereiche innerhalb des Produkts, wobei für die Analyse mehrere Analysemodule bzw.

Analyseschritte auf der Grundlage verschiedener Analysekriterien durchgeführt werden. Jeder Analyseschritt liefert dabei einen Output, der mit den gespeicherten Lebensmittelproduktdaten verglichen wird.

So können Lebensmittelprodukte automatisch und verlässlich bestimmt und sortiert werden.

Für eine besonders sichere Erkennung wird die Analyse in mindestens fünf Modulen bzw. Analyseschritten durchgeführt. In einer Ausführungsart werden zehn bis fünfzehn (oder mehr) Module durchgeführt, die entsprechend viele Kriterien liefern. (Die Anzahl der Kriterien kann größer sein als die Anzahl der durchgeführten Module.) So ist es möglich, Lebensmittelprodukte mit einer hierarchischen Methode zu bestimmen bzw. zu sortieren. Bei einer hierarchischen Methode werden nicht-übereinstimmende Lebensmittel so lange eliminiert, bis eine Entsprechung gefunden wird.

Bei den Outputs handelt es sich um numerische Werte, die eine Evaluierungsvorrichtung liefert. Das Evaluierungsgerät ermittelt diese numerischen Outputwerte vor allem durch die Bildverarbeitung, also einer Verarbeitung optischer Daten.

Die Analyse besteht beispielsweise aus einer Farbanalyse und einer Oberflächenanalyse des Lebensmittelprodukts.

Die Analyseschritte basieren hauptsächlich auf optischen Daten. Die optischen Daten basieren auf den Bilddaten der von der/den Kamera(s) aufgenommenen Bilder.

In einem weiteren Verfahrensschritt können die ermittelten Daten an eine Anzeigevorrichtung und/oder eine Etikettiervorrichtung und/oder eine Sortiervorrichtung weitergegeben werden, um die Daten der Lebensmittelprodukte automatisch anzuzeigen, oder die Lebensmittelprodukte automatisch zu etikettieren oder zu sortieren.

### KURZE BESCHREIBUNG DER ZEICHNUNGEN

- Figur 1: zeigt eine schematische Ansicht einer Ausführungsart eines Kontrollsystems für Lebensmittel gemäß der vorliegenden Erfindung;
- Figur 2: zeigt einen Querschnitt entlang der Linie 2-2 der Figur 1 einer Kontroll-Station;
- Figur 3: zeigt eine Ansicht eines Positionierungsbereichs der Kontroll-Station von Figur 2;
- Figur 4: zeigt einen schematischen Querschnitt einer versiegelten Schale (Schale mit Deckel) einschließlich eines Lebensmittelprodukts;
- Figur 5: zeigt ein schematisches Ablaufdiagramm, das eine Ausführungsart der Lebensmittelproduktidentifizierung und -sortierung illustriert;
- Figur 6: zeigt ein schematisches Diagramm zur Illustration einer Farbanalyse; das Diagramm hat als Achsen die Farben Rot, Grün und Blau und ein analysiertes Lebensmittelprodukt ist mit Punkten dargestellt;
- Figur 7: zeigt ein Bild eines verpackten Fleischprodukts während eines Analyseschritts; das Originalbild hat blaue, rote und grüne Bereiche. Es ist möglich, zwischen mageren Bereichen, fetten Bereichen und lasergenerierten Lichtbereichen zu unterscheiden;
- Figur 8(a) bis (e): zeigen verschiedene Beispiele von Lebensmittelprodukten und einer lasergenerierten Lichtlinie; und
- Figur 9: zeigt eine schematische Ansicht eines Lebensmittelprodukts und verschiedene lasergenerierte Lichtlinien, mit Hilfe derer eine Verpackungsanalyse möglich ist.

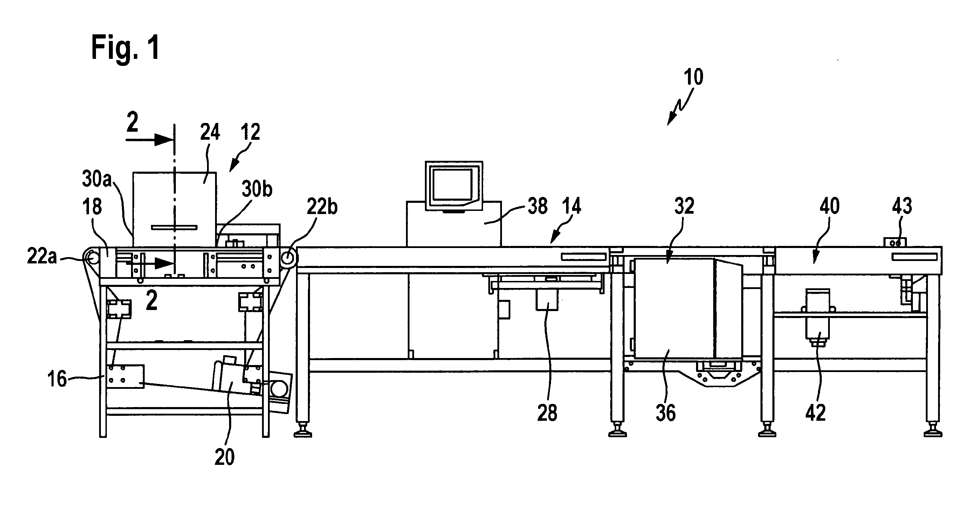

Eine Ausführungsart eines Kontrollsystems für Lebensmittel, in Figur 1 mit Ziffer 10 bezeichnet, umfasst eine optische Kontroll-Station 12 bzw. Vision-Station 12 zur Durchführung von optischen Kontrollen an Lebensmitteln. Das Kontrollsystem für Lebensmittel 10 umfasst außerdem eine Wägestation 14 zum Wiegen von Lebensmittelprodukten (zusammen mit ihrer Verpackung). Die Kontroll- oder Vision-Station 12 und die Wägestation 14 können so verbunden werden, dass während der optischen Kontrolle der Lebensmittelprodukte diese auch gewogen werden oder sie können so getrennt werden, dass die Wägestation 14 hinter der Kontroll-Station 12 folgt (siehe Fig. 1).

Die Kontroll-Station 12 umfasst einen Ständer 16. Auf diesem Ständer 16 verläuft ein Förderband 18 für den Transport von Lebensmittelprodukten durch die Vision-Station 12 (zur Wägestation 14). Das Förderband 18 wird von einem Motor 20 angetrieben, der auf dem Ständer 16 befestigt ist.

Darüber hinaus sind die Rollen 22a und 22b auf dem Ständer 16 zum Führen des Förderbands 18 befestigt.

Die optische Kontroll-Station 12 umfasst ein Gehäuse 24, das auf dem Ständer 16 angebracht ist. In diesem Gehäuse 24, werden die Lebensmittelprodukte, wie unten beschrieben, beleuchtet.

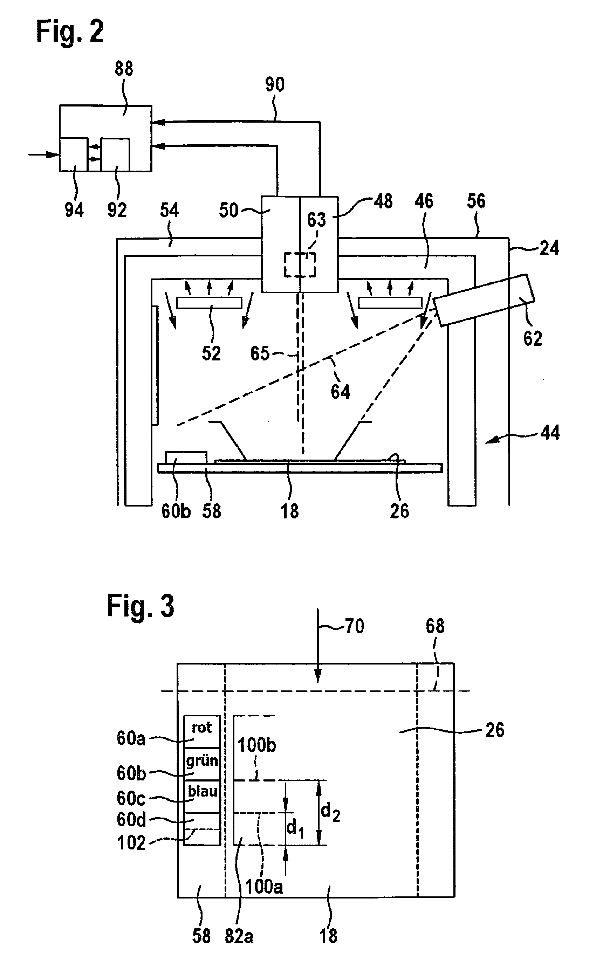

Die zu kontrollierenden Lebensmittelprodukte werden, wie weiter unten beschrieben, in Schalen verpackt. Die Kontroll-Station 12 umfasst einen Positionierungsbereich und insbesondere Schalenpositionierungsbereich 26 (Fig. 2 und 3) in dem Schalen mit Lebensmittelprodukten während der Überprüfung positioniert werden.

Die Lebensmittelprodukte können entweder bei still stehendem oder bei durchgehend laufendem Förderband 18 geprüft werden. In letzterem Fall kann der Schalenpositionierungsbereich 26 ein Bereich sein, der sich mit dem Förderband 18 bewegt.

Die Wägestation 14, die Waagen 28 umfasst, ist so angeordnet, dass das Gewicht der Schale-Lebensmittelprodukt-Einheit entweder während oder nach der optischen Kontrolle derselben gemessen werden kann.

Das Gehäuse 24 verfügt über Öffnungen 30a, 30b, die einander gegenüber liegen und eine Durchführung der Lebensmittelprodukte durch die Kontroll-Station 12 erlauben. Das Gehäuse 24 ist damit in der Form eines Tunnels ausgeführt.

Das Kontrollsystem für Lebensmittel umfasst weiterhin eine Etikettierstation 32, die auf die Wägestation 14 folgt. Die optische Kontroll- bzw. Vision-Station 12 und die Etikettierstation 32 sind durch eine Transportstation zum Transport der Lebensmittel von der Vision-Station 12 zur Etikettierstation 32 verbunden.

An der Etikettierstation 32 werden die Schalen mit dem Lebensmittelprodukt etikettiert. Die Etiketten, bei denen es sich z. B. um gedruckte Etiketten handelt, werden an der Etikettierstation 32 mit den Lebensmittelproduktinformationen versehen, die aus den Daten der Vision-Station 12 und der Wägestation 14 ermittelt wurden. Auf diese Weise können die Etiketten Informationen zum Gewicht enthalten und den Verkaufspreis ausweisen, der aus den von der Wägestation 14 ermittelten Gewichtsinformationen und den über die Vision-Station 12 ermittelten Produkterkennungsdaten berechnet wurde.

Die Etikettierstation 32 kann z. B. ein Etikettiergerät 36 umfassen, das die Etiketten über einen Stempel, via Luftstrom oder über eine sich drehende Walze auf der Lebensmittelverpackung anbringt.

Das Kontrollsystem für Lebensmittel 10 umfasst weiterhin ein Steuergerät 38 zur Steuerung der Vision-Station 12, der Wägestation 14 und der Etikettierstation 32. Das Steuergerät 38 berechnet beispielsweise die Verkaufspreise in Anlehnung an die Daten der Vision-Station 12 und der Wägestation 14.

Darüber hinaus kann eine Kontrollstation 40 nach der Etikettierstation 32 angeordnet werden. Die Kontrollstation 40 umfasst z. B. (mindestens) eine Kamera 42. Mit dieser Kamera 42 kann die Kontrollstation 40 prüfen, ob ein Etikett auf der Lebensmittelverpackung vorhanden ist und ob das Etikett richtig platziert ist. Außerdem kann mit der Kamera 42 kontrolliert werden, ob die gedruckten Etiketteninformationen richtig sind, wenn die Bilddaten der Kamera 42 entsprechend vom Steuergerät 38 analysiert werden. (Das Steuergerät 38 könnte z. B. OCR/OCV verwenden.)

Die Kontrollstation 40 kann eine Schubvorrichtung 43 zum Entfernen von verpackten Lebensmittelprodukten, die als nicht bestimmten Qualitätsstandards entsprechend eingestuft wurden, umfassen (z. B. durch Messergebnisse der Vision-Station 12).

Die Kontroll- bzw. Vision-Station 12 umfasst eine Haltevorrichtung 44 (Fig. 2), die auf dem Ständer 16 platziert ist. Die Haltevorrichtung 44 kann z. B. eine Art Gestell sein.

Die Haltevorrichtung umfasst eine Stange 46, die über dem Schalenpositionierungsbereich 26 in einem bestimmten Abstand positioniert ist. Der Arm 46 hält eine erste Kamera 48 und ggf. eine zweite Kamera 50. Das Sichtfeld der ersten Kamera 48 und der zweiten Kamera 50 ist der Schalenpositionierungsbereich 26.

Bei den Kameras 48 und 50 handelt es sich um handelsübliche Kameras.

Die Kameras 48 und 50 sind innerhalb des Gehäuses 24 platziert. Die Haltevorrichtung 44 kann sich entweder ganz, zum Teil innerhalb oder auch komplett außerhalb des Gehäuses 24 befinden.

Innerhalb des Gehäuses 24 werden Lichtquellen 52 für separates rotes, grünes und blaues Licht angeordnet. Die Lichtquellen 52 können beispielsweise Leuchtdioden sein. Komplett an allen Wänden oder nur teilweise an den Wänden des Gehäuses 24 befindet sich ein Reflexionsbereich 54. Ein Reflexionsbereich 54 entsteht z. B. auf einer Seite 56 des Gehäuses 24, die in Richtung des Schalenpositionierungsbereichs 26 zeigt und über diesem liegt. Der Reflexionsbereich 54 kann beispielsweise aus Platten gebildet werden. Die Platten können aus Plastik sein.

Das Licht aus den Lichtquellen 52 strahlt in Richtung des Reflexionsbereichs 54 und wird von dort in Richtung des Schalenpositionierungsbereichs 26 reflektiert. Das Licht aus den Lichtquellen 52 vermischt sich auf dem Weg in Richtung des Reflexionsbereichs 54 und wenn es von diesem reflektiert wird. Dadurch wird der Schalenpositionierungsbereich 26 mit weißem Licht beleuchtet. In diesem Sinne kann der Reflexionsbereich 54 als eine Quelle für weißes Licht gesehen werden, die den Schalenpositionierungsbereich 26 beleuchtet. Die Lichtquellen 52 und der Reflexionsbereich 54 werden so gebildet und angeordnet, dass der Schalenpositionierungsbereich 26 gleichmäßig beleuchtet ist.

Anstelle von "separaten" Quellen für einzelne Farben kann auch eine Breitbandquelle oder Breitbandquellen für weißes Licht verwendet werden. Idealerweise werden eine oder mehrere Breitband-Leuchtdioden verwendet. Prinzipiell ist es auch möglich, andere Breitband-Lichtquellen, wie Halogenlampen, zu verwenden.

Auf dem Ständer 26 wird eine Halteplatte 58 angebracht. Die Halteplatte 58 wird innerhalb des Gehäuses 24 angebracht. Das Förderband 18 wird über die Halteplatte 58 geführt.

Auf der Halteplatte 58 werden Referenzfelder 60a, 60b, 60c, 60d angeordnet. Das Referenzfeld 60a ist ein rotes Farbreferenzfeld. Das Referenzfeld 60b ist ein grünes Farbreferenzfeld und das Referenzfeld 60c ist ein blaues Farbreferenzfeld. Das Referenzfeld 60d ist ein weißes Farbreferenzfeld. Die Farben der Referenzfelder entsprechen den Farben des Lichts, das von der entsprechenden Lichtquelle abgegeben wird. Das heißt, die Farbe des Referenzfelds 60a entspricht der Farbe der Lichtquellen, die rotes Licht abgeben, die Farbe des Referenzfelds 60b entspricht der Farbe der Lichtquellen, die grünes Licht abgeben und die Farbe des Referenzfelds 60c entspricht der Farbe der Quellen für blaues Licht.

Das Referenzfeld 60d für die weiße Farbe erlaubt den Ausgleich auf ein weißes Ziel.

Die Referenzfelder 60a, 60b, 60c werden innerhalb der Sichtweite der ersten Kamera 48 angeordnet (und optional der zweiten Kamera 50). Die Referenzfelder 60a, 60b, 60c werden so am oder in der Nähe des Schalenpositionierungsbereichs 26 angeordnet, dass sich sowohl die Referenzfelder 60a, 60b, 60c als auch das Lebensmittelprodukt im Schalenpositionierungsbereich 26 innerhalb des Sichtfelds der ersten Kamera 48 befinden.

Über die Referenzfelder 60a, 60b und 60c können Leuchtdichte und Farbbalance der Kameras 48 und 50 überwacht werden. Die Einstellungen der Kameras 48 und 50 können ggf. automatisch angepasst werden.

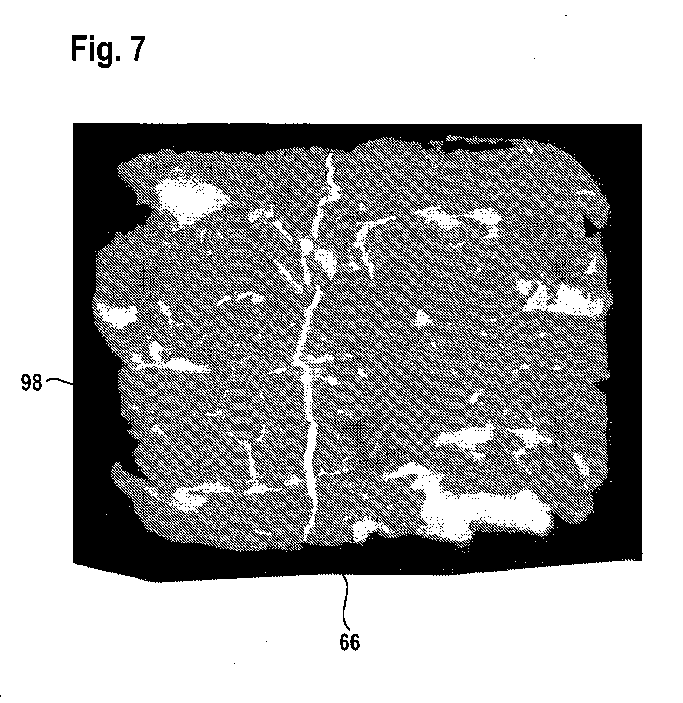

Die Haltevorrichtung 44 enthält außerdem ein Lasergerät 62, das die Quelle für grünes Laserlicht 64 ist. Das Lasergerät 62 generiert eine (grüne) lasergenerierte Lichtlinie 66 (Fig. 7) über dem verpackten Lebensmittelprodukt. Diese Lichtlinie 66 wird für verschiedene Analyse- und Evaluierungsschritte verwendet, die weiter unten beschrieben werden.

Das Lasergerät 62 gibt (über eine entsprechende optische Anlage) einen scannenden Lichtfächer ab, der schräg auf eine Ebene 68 trifft, die senkrecht zur Halteplatte 58 und dem Förderband 18 steht. Die Ebene 68 steht außerdem senkrecht zu einer Transportrichtung 70 von Lebensmitteln durch die Vision-Station 12. So ist, wie unten beschrieben, eine Verpackungsanalyse möglich.

Ein weiteres Lasergerät 63 kann zum Scannen in einer Linie, die im Wesentlichen parallel zur Transportrichtung 70 verläuft, verwendet werden. Dieses Lasergerät gibt einen scannenden Lichtfächer ab, der in Figur 2 mit der Ziffer 65 gekennzeichnet ist. Dieser Lichtfächer 65 kann parallel oder in einem Winkel zu einer Ebene stehen, die senkrecht zur Ebene 68 verläuft.

Das Lasergerät 63 gibt Laserlicht in derselben Farbe ab, wie das Lasergerät 62.

Es können alternativ auch mehrere Lasergeräte verwendet werden, die scannende Lichtfächer abgeben,

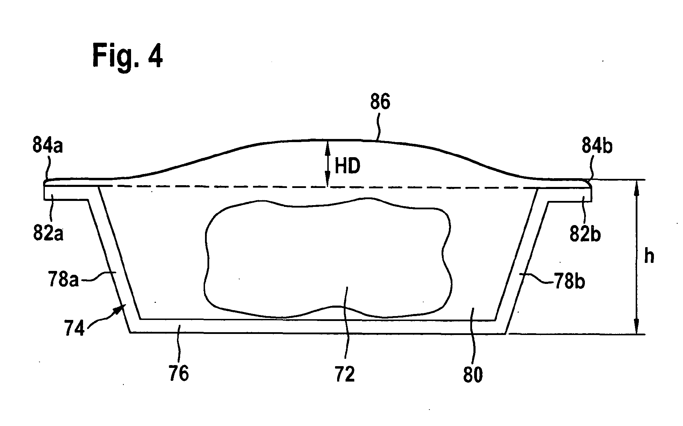

Wie in Figur 4 gezeigt, sind die zu untersuchenden Lebensmittelprodukte 72 in entsprechenden Schalen 74 verpackt. Die Schale 74 hat z. B. ein Unterteil 76 und Wandteile 78a, 78b. Über das Unterteil 76 und die Wandteile 78a und 78b wird ein Aufnahmebereich 80 für das Lebensmittelprodukt 72 gebildet.

Die Schale 74 umfasst außerdem auf den Wandteilen 78a und 78b flanschähnliche Erweiterungen 82a und 82b, die parallel zum Unterteil 76 liegen. Diese Erweiterungen 82a und 82b bieten Versiegelungsbereiche 84a und 84b, an denen ein Deckel 86 angebracht werden kann. Beim Deckel 86 handelt es sich um eine durchsichtige folienähnliche Abdeckung, die einen optischen Zugriff auf das Lebensmittelprodukt 72 im Aufnahmebereich 80 erlaubt.

Die Versiegelung 86 bildet eine Art Haube auf der Schale 74. Der Deckel 86 ist so geformt, dass das Lebensmittelprodukt 72 von äußeren Einflüssen geschützt ist. Es ist möglich, unter dem Deckel 86 ein schützendes Gas zum Schutz des Lebensmittelprodukts 72 einzuschließen.

Es ist vorteilhaft, wenn sich die Schale 74 vom Lebensmittelprodukt in der Schale abhebt. Die Schale 74 sollte vor allem, wenn es sich beim Lebensmittelprodukt um verarbeitetes Fleisch handelt, nicht rot oder weiß sein. Idealerweise ist die Schale 74 schwarz.

Das Kontrollsystem für Lebensmittel 10 umfasst ein Evaluierungsgerät 88 (Fig. 2) zur Analyse der von der ersten Kamera 48, und optional zweiten Kamera 50, aufgenommenen Bilder. Das Evaluierungsgerät 88 kann Teil des Steuergeräts 38 sein oder auch davon getrennt, aber damit verbunden.

Das Evaluierungsgerät 88 empfängt über entsprechende Signale 90 die Pixeldaten (Bilddaten) der Kameras 48 und 50. Das Evaluierungsgerät 88 verfügt über einen Datenspeicher 92 zum Speichern der Lebensmittelproduktdaten. Durch den Vergleich der analysierten und evaluierten Bilddaten mit den gespeicherten Daten, kann ein Lebensmittelprodukt bestimmt und/oder sortiert werden.

Darüber hinaus verfügt das Evaluierungsgerät 88 über einen Lernbereich 94 zur Durchführung von Lernprozessen und zum Einlesen der Daten von Lebensmittelprodukten, die noch nicht gespeichert sind.

Das Kontrollsystem für Lebensmittel 10 und besonders die optische Kontroll-bzw. Vision-Station 12 funktioniert wie folgt:

Ein in einer Schale 74 verpacktes Lebensmittelprodukt wird in den Schalenpositionierungsbereich 26 platziert. Für die optische Analyse des Lebensmittelprodukts kann die Schale still stehen oder in Bewegung sein. Auf diese Weise ist es möglich, eine Überwachung bei still stehender Schale oder eine kontinuierliche Überwachung durchzuführen.

Die Lichtquellen 52 produzieren rotes, grünes und blaues Licht. Der Schalenpositionierungsbereich 26 wird mit weißem Breitbandlicht vom Aufnahmebereich 80 beleuchtet. Die Spiegelung auf einem Deckel 86 kann über die Reflexion des Lichts vom Aufnahmebereich 80 minimiert werden.

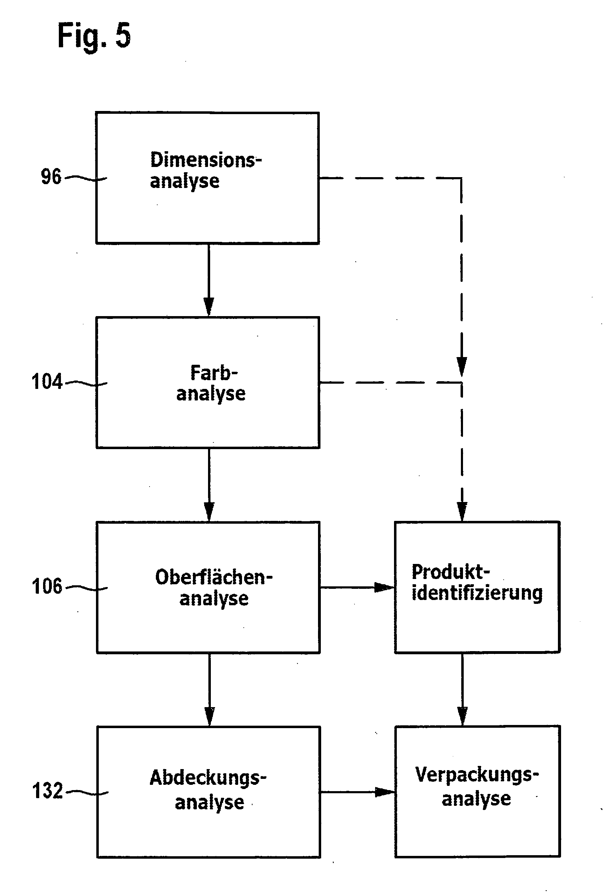

Die Kamera 48 wird auf einen Schalenrand ausgerichtet und ausgelöst. Das Evaluierungsgerät 88 ermittelt die Größe der Schale. Daraufhin werden über die Bildverarbeitung die Kontur oder die Form des Lebensmittelprodukts 72 in der Schale 74 analysiert. In Abbildung 5 wird dies mit dem Kasten 96 "Größenanalyse" gezeigt.

Ist die Kontur/Form des Lebensmittelprodukts 72 bekannt, kann das Evaluierungsgerät 88 (in einem Bild) das Lebensmittelprodukt 72 von der Schale 74 unterscheiden.

Figur 7 zeigt ein Bildbeispiel eines Lebensmittelprodukts 98 (Fleisch), das vom Evaluierungsgerät 88 nach der Trennung von Lebensmittelprodukt 98 und Schale 74 generiert wird.

Die lasergenerierte Lichtlinie 66 verläuft über dem Lebensmittelprodukt 72 und über den Erweiterungen 82a, 82b auf der Schale 74. Das Evaluierungsgerät 88 sucht nach dem entsprechenden Teil 100a, 100b der Lichtlinien 66 auf den Erweiterungen 82a, 82b. Außerdem evaluiert das Evaluierungsgerät 88 die Lichtlinie 102 außerhalb der Schale 74. Es wird z. B. die Lichtlinie auf den Referenzfeldern 60a, 60b, 60c evaluiert.

Zwischen der Lichtlinie 102 und dem Teil 100a, 100b gibt es einen Versatz d1, d2 abhängig von der Höhe h (Fig. 4) der Schale 74. Je größer dieser Versatz ist desto höher ist die Schale.

Figur 3 zeigt zwei Beispiele: Ein geringer Versatz d1 und ein größerer Versatz d2. Die entsprechenden Schalen sind unterschiedlich hoch, wobei die für den Versatz d2 verantwortliche Schale höher ist als die andere Schale. Das Evaluierungsgerät 88 kann den Versatz d1, d2 über die Bildverarbeitung ermitteln. Anhand dieses Versatzes kann die Schalenhöhe ermittelt werden.

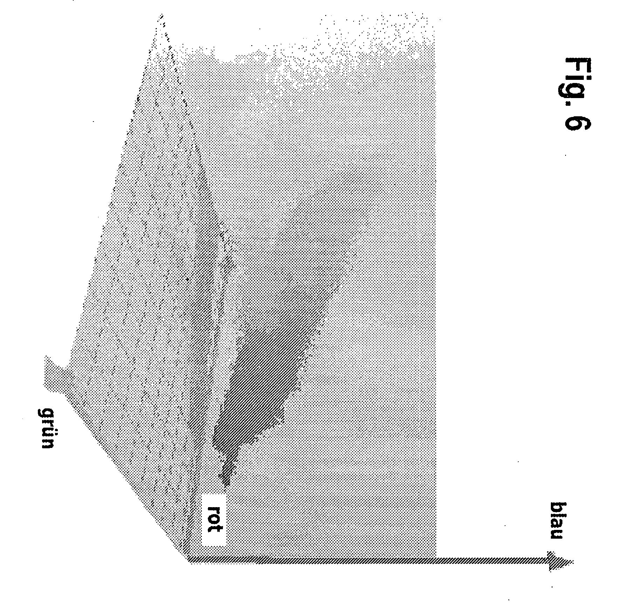

Das Evaluierungsgerät 88 führt eine Farbanalyse der Bilder der ersten Kamera 48 durch. Dies wird in Figur 5 mit dem Kasten 104 gezeigt. Für die Farbanalyse werden die Pixel der Bilder von der ersten Kamera 48 in rote, grüne und blaue Farbbereiche eingeteilt (siehe Fig. 6). Durch eine Farbanalyse können verschiedene Bereiche im Lebensmittelprodukt unterschieden werden. Bei Fleisch können z. B. Knochenbereiche, magere und fette Bereiche unterschieden werden. Außerdem ist es mit der Farbanalyse möglich, einen Laserlichtbereich für die lasergenerierte Lichtlinie 66 zu unterscheiden. Dieser wird zur weiteren Analyse verwendet.

Figur 7 zeigt ein Bild eines Fleischprodukts nach einer Farbanalyse. Das Originalbild ist eine blaue Falschfarbendarstellung, einschließlich blauer Bereiche, roter Bereiche und auch eines grünen Bereichs für die lasergenerierte Lichtlinie 65. Das Bild ist segmentiert und ermöglicht eine Unterscheidung von mageren und fetten Bereichen.

Des Weiteren wird eine Oberflächenanalyse durchgeführt (siehe Fig. 5, Kasten 106). Für die Oberflächenanalyse wird die lasergenerierte Lichtlinie 66 verwendet.

Das Evaluierungsgerät 88 analysiert die lasergenerierte Lichtlinie 66 auf dem Lebensmittelprodukt 72. Dafür werden die entsprechenden grünen Pixel in den aufgenommenen Bildern evaluiert.

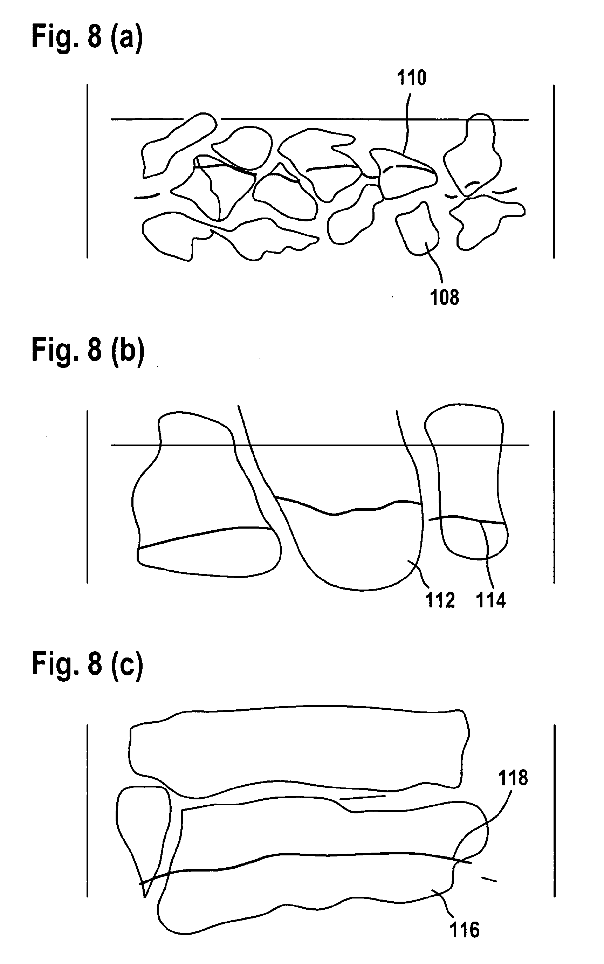

Wie aus den Figuren 8(a) bis 8(e) hervor geht kann die Lichtlinie 66 abhängig von dem Lebensmittelprodukt versetzt sein.

Handelt es sich beim Lebensmittelprodukt (z. B. bei Fleisch) um Fleisch für Eintopf 108, ist der entsprechende Laserstrahl 110 fragmentiert (siehe Figur 8(a)). Zwischen den verschiedenen den verschiedenen Teilen des Fleisches für Eintopf gibt es einen Versatz.

Falls es sich beim Lebensmittelprodukt um mehrere geschnittene Fleischscheiben 112 handelt (siehe Figur 8(b)), ist die entsprechende Lichtlinie 114 weniger fragmentiert, kann aber einen größeren Versatz zwischen den benachbarten geschnittenen Stücken aufweisen.

Handelt es sich um ein einziges Stück 116 (siehe Figur 8(c)), ist die entsprechende Lichtlinie 118 nicht fragmentiert.

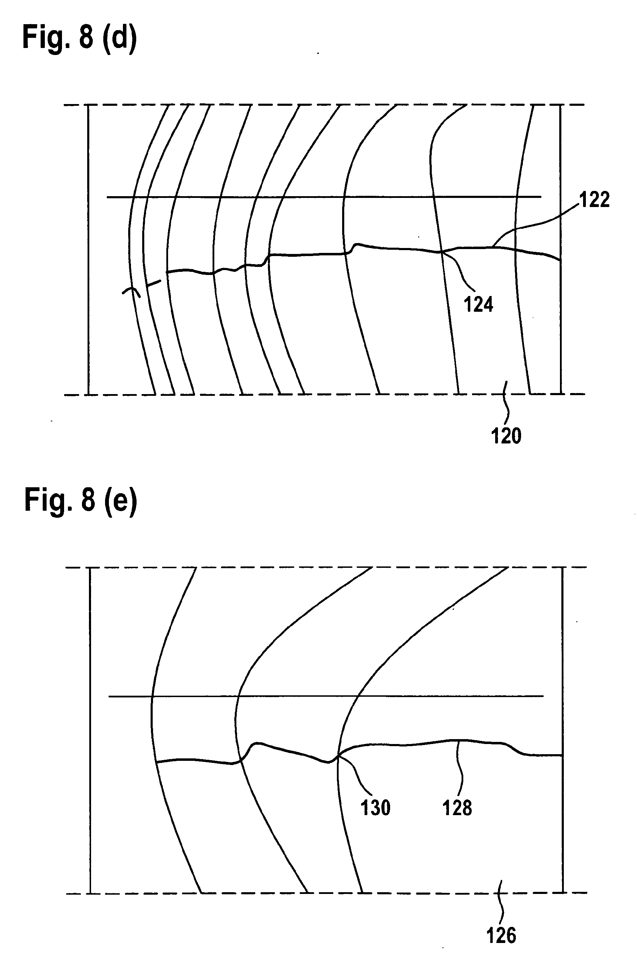

Über die Analyse der Lichtlinie ist es außerdem möglich, zwischen dicken und dünnen Scheiben des Lebensmittelprodukts zu unterscheiden. Figur 8(d) zeigt eher dünne Scheiben 120. Die Lichtlinie 122 hat einen Versatz 124, der auf unterschiedliche Scheiben hinweist. Bei dünnen Scheiben 120 ist der Versatz geringer als bei dicken Scheiben 126 (siehe Figur 8(e). Hier weist die entsprechende Lichtlinie 128 einen Versatz 130 am Übergang zu den verschiedenen Scheiben auf. Die Lichtlinie 128 ist außerdem weniger segmentiert als die Lichtlinie 122, was darauf hinweist, dass weniger Scheiben vorhanden sind.

Anhand der Farbinformationen der Farbanalyse und der Daten aus der Oberflächenanalyse können die Lebensmittelprodukte bestimmt und/oder sortiert werden.

Es kann außerdem eine Deckelanalyse durchgeführt werden (siehe Figur 5, Kasten 132). Diese kann beispielsweise vor der Farbanalyse oder nach der Oberflächenanalyse durchgeführt werden.

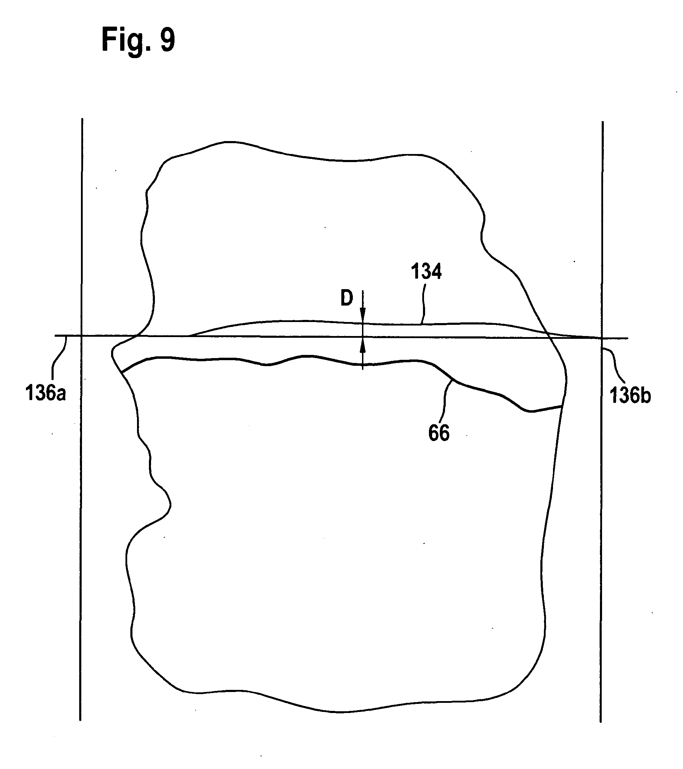

Ein folienähnlicher Deckel 86 ist für eine weitere lasergenerierte Lichtlinie 134 (Fig. 9) verantwortlich. Diese Lichtlinie 134 entsteht durch Laserlichtreflexion auf dem Deckel 86. Durch den Vergleich des Versatzes D (Fig. 9) zwischen der Lichtlinie 134 und den Lichtlinienteilen 136a, 136b auf den Erweiterungen 82a, 82b der Schale 74 ist es möglich, die Höhe der Folienhaube zu bestimmen (siehe HD in Figur 4). So kann festgestellt werden, ob der Deckel 86 das Lebensmittelprodukt berührt. Es ist außerdem möglich, die Position einer Folienhaube (wie von der Lichtlinie 134 erkannt) mit der Position des Produkts in seiner Verpackung zu vergleichen. Decken sich die beiden Positionen, berührt der Deckel 86 das Produkt.

Mit der Analyse der Lichtlinienteile 136a, 136b auf den Erweiterungen 82a, 82b kann festgestellt werden, ob sich Einschlüsse in den Versiegelungsbereichen 84a, 84b befinden. Es könnten sich z. B. Teile des Lebensmittelprodukts zwischen dem Deckel 86 und den Erweiterungen 82a, 82b befinden. Das Evaluierungsgerät 88 analysiert über die Bildverarbeitung, ob die Versiegelungsbereiche 84a, 84b dieselbe Farbe haben wie die Schale (z. B. schwarz). Haben sie eine andere Farbe, muss sich etwas im Versiegelungsbereich unter oder im Deckel 86 befinden.

Wie bereits erwähnt, kann die Verpackungsanalyse anhand der Deckelanalyse vor der Farbanalyse durchgeführt werden.

In einer besonderen Ausführungsart erfolgt die Kontrolle von Lebensmittelprodukten zur Identifizierung und Sortierung wie folgt:

Die erste Kamera 48 wird auf einen Rand einer Schale 74 ausgerichtet und ausgelöst. Das Evaluierungsgerät 88 sucht nach Lichtlinienteilen auf beiden Erweiterungen 82a, 82b, um die Schalenhöhe H zu berechnen. Anschließend wird die Lichtlinie 134 auf dem Deckel 86 gesucht (via Bildverarbeitung im Evaluierungsgerät 88 anhand der Pixeldaten der ersten Kamera 48). Mit Hilfe der entsprechenden Informationen wird die Höhe HD der Rundung des Deckels berechnet.

Außerdem werden die Parameter der Schale 74, d. h. die Erweiterungen 82a, 82b, auf mögliche Einschlüsse geprüft. Dabei wird nach Farbpixeln gesucht, die es ohne Einschlüsse nicht gäbe.

Das Evaluierungsgerät 88 ermittelt daraufhin die Kontur oder Form des Lebensmittelprodukts 82 und teilt das Lebensmittelprodukt in unterschiedliche Farbbereiche. Das Bild des Lebensmittelprodukts 72 wird dabei z. B. in Laserbereiche und magere und fette Bereiche eingeteilt.

Das Bild des Lebensmittelprodukts 72 innerhalb seiner Konturen wird anhand von verschiedenen Analysemodulen analysiert. Das Ergebnis der Analyse ist ein numerischer Output für jedes Analysemodul. Es können ggf. zehn bis fünfzehn (oder mehr) verschiedene Analysemodule durchgeführt werden. Beispiele für Analysemodule sind:

Die Textur des Lebensmittelprodukts um eine lasergenerierte Lichtlinie 66. (Mit einer solchen Analyse ist es beispielsweise möglich, zwischen geschnittenem Fleisch und gewürfeltem Fleisch zu unterscheiden).

Weitere Beispiele sind: die Anzahl der Lichtliniensegmente, die Längenverteilung von Lichtliniensegmenten, die Winkelverteilung von Lichtliniensegmenten oder der Versatz zwischen Lichtliniensegmenten. Darüber hinaus wird die Form der Lebensmittelproduktkontur analysiert. Hieraus können sich verschiedene Kriterien ergeben. Außerdem wird die Größenverteilung von Fettbereichen analysiert. Dies kann durch Bildverarbeitung und Zerlegung des Bildes in Rechteckbereiche erfolgen Ein weiteres Kriterium ist die Formverteilung von Fettbereichen. Dies kann ebenfalls durch Bildverarbeitung und Zerlegung des Bildes in Rechteckbereiche erfolgen.

Die vom Evaluierungsgerät 88 berechneten Outputwerte werden zur Eliminierung von nicht übereinstimmenden Lebensmitteln verwendet. Die entsprechenden Daten der Lebensmittelprodukte werden im Datenspeicher 92 gespeichert. Wird eine Übereinstimmung gefunden, gibt das Steuergerät 38 die Informationen weiter an das Etikettiergerät 36.

In einer Ausführungsart in Anlehnung an die vorliegende Erfindung werden nicht übereinstimmende Lebensmittelprodukte so lange verworfen bis ein übereinstimmendes Lebensmittelprodukt übrig ist.

Mit der zweiten, optionalen, Kamera 50 kann eine Teilaufnahme nur des lasergenerierten Lichtlinienbereichs gemacht werden, während das Lebensmittelprodukt durch die Vision-Station 12 geführt wird. So kann z. B. die zweite Kamera 50 Bilddaten liefern, anhand derer erkannt werden kann, ob der Deckel 86 das Lebensmittelprodukt berührt. Das Evaluierungsgerät 88 kann daraufhin ein entsprechendes Signal geben, das zur Folge hat, dass die entsprechende Schale bei der Etikettierung an der Etikettierstation 32 ignoriert und die entsprechende Verpackung beim Ausstoß verworfen wird.

Die zweite Kamera 50 überwacht außerdem die Referenzfelder 60a, 50b, 60c.

Das Lernmodul 94 kann ein intelligentes Lernmodul oder ein Setupmodul sein. Beim Führen eines neuen Produkts durch die Vision-Station 12 werden die entsprechenden Outputwerte für jedes der oben genannten Module berechnet. Das Lernmodul 94 kann prüfen, ob die Eigenschaften eines Lebensmittelprodukts mit bereits gespeicherten Produktdaten übereinstimmen oder darauf hinweisen, dass es sich um ein völlig neues Produkt handelt. Es können auch Bereichseinstellungen für jedes Kriterium vorgeschlagen werden.

## Patentansprüche

1. Kontrollvorrichtung für Lebensmittel zur Identifizierung und/oder zur Sortierung von in einer Schale verpackten Lebensmittelprodukten, umfassend einen Positionierungsbereich, eine Beleuchtungsvorrichtung für die Beleuchtung des Lebensmittelprodukts mit weißem Licht, mindestens eine Kamera für die Aufnahme von Bildern des beleuchteten Lebensmittelprodukts und eine Evaluierungsvorrichtung für die Bildverarbeitung, wobei die Evaluierungsvorrichtung eine Erkennung der Lebensmittelprodukte mittels einer Farbanalyse und/oder einer Helligkeitsanalyse der von der Kamera aufgenommenen Bilder durchführt, **dadurch gekennzeichnet, dass** die Evaluierungsvorrichtung über einen Konturfilter oder Kantenfilter die Kontur oder Form des Lebensmittelprodukts in der Schale erkennt und so die Form des Lebensmittelprodukts von der Schale trennt und über einen Vergleich mit Solldaten einer fehlerfreien Verpackung Einschlüsse in einer Versiegelung erkennt.

2. Kontrollvorrichtung für Lebensmittel nach Anspruch 1, **dadurch gekennzeichnet, dass** die Beleuchtungsvorrichtung Lichtquellen für rotes, grünes und blaues Licht umfasst, die das Licht in Richtung eines das ausgestrahlte Licht zu weißem Licht mischenden Reflexionsbereiches ausstrahlen.

3. Kontrollvorrichtung für Lebensmittel nach Anspruch 2, **dadurch gekennzeichnet, dass** eine Lichtquelle mindestens eine Leuchtdiode aufweist, wobei die Leuchtdiode rotes oder grünes oder blaues oder weißes Licht ausstrahlt und vorzugsweise ein Polarisationsfilter zum Polarisieren des ausgestrahlten Lichts vorgesehen ist.

4. Kontrollvorrichtung für Lebensmittel nach einem der Ansprüche 1 bis 3, **dadurch gekennzeichnet, dass** der Positionierungsbereich ein Gehäuse aufweist, welches über Öffnungen zum Durchführen von Lebensmittelprodukten und/oder Schalen verfügt und zum Transport der Lebensmittelprodukte und/oder Schalen ein Förderband vorgesehen ist.

5. Kontrollvorrichtung für Lebensmittel nach einem der Ansprüche 1 bis 4, **dadurch gekennzeichnet, dass** Farbreferenzfelder auf dem oder in der Nähe des Positionierungsbereichs innerhalb der Sichtweite mindestens einer ersten Kamera angeordnet sind, wobei vorzugsweise ein Farbreferenzfeld für jede der Farben Rot und/oder Grün und/oder Blau und/oder Weiß verwendet wird.

6. Kontrollvorrichtung für Lebensmittel nach einem der Ansprüche 1 bis 5, **dadurch gekennzeichnet, dass** die Evaluierungsvorrichtung zum Erkennen von fetten Bereichen und/oder mageren Bereichen und/oder Knochenbereichen bei Fleisch- oder Wurstprodukten ausgebildet ist, indem sie ein Bild im Hinblick auf die Positionen der Farben Rot, Grün und Blau und vorzugsweise deren Helligkeit analysiert.

7. Kontrollvorrichtung für Lebensmittel nach einem der Ansprüche 1 bis 6, **dadurch gekennzeichnet, dass** die Evaluierungsvorrichtung die Form und/oder Größe der Lebensmittelprodukte in der Schale, und/oder die Form und/oder Größe der Schalen und/oder eine Formverteilung und/oder die Größe von fetten Bereichen und/oder mageren Bereichen und/oder Knochenbereichen des Lebensmittelprodukts erkennt.

8. Kontrollvorrichtung für Lebensmittel nach einem der Ansprüche 1 bis 7, **dadurch gekennzeichnet, dass** sie ein Lasergerät (62) umfasst, das mindestens eine lasergenerierte Lichtlinie über der Schale produziert, indem es vorzugsweise grünes Licht in einem Winkel zu einer Ebene senkrecht zum Positionierungsbereich abgibt.

9. Kontrollvorrichtung für Lebensmittel nach Anspruch 8, **dadurch gekennzeichnet, dass** die Evaluierungsvorrichtung lasergenerierte Lichtlinien in einem aufgenommenen Bild erkennt und im Hinblick auf Segmentierung analysiert, wobei sie insbesondere die Anzahl der Segmente, und/oder die Länge der Segmente, und/oder die Längenverteilung der Segmente, und/oder die Winkel der Segmente, und/oder die Winkelverteilung der Segmente und/oder den Versatz zwischen den Segmenten bestimmt.

10. Kontrollvorrichtung für Lebensmittel nach Anspruch 8 oder 9, **dadurch gekennzeichnet, dass** die Evaluierungsvorrichtung die Textur des Lebensmittelprodukts im Bereich einer lasergenerierte Lichtlinie analysiert.

11. Kontrollvorrichtung für Lebensmittel nach einem der Ansprüche 8 bis 10, **dadurch gekennzeichnet, dass** die Evaluierungsvorrichtung die Höhe der Rundung eines Deckels der Schale durch Vergleich der Reflexionslichtlinie einer lasergenerierte Lichtlinie, die durch Lichtreflexion auf dem durchsichtigen versiegelten Deckel zustande kommt, mit dem Licht, das an einem Schalenrand reflektiert wird, bestimmt.

12. Kontrollvorrichtung für Lebensmittel nach einem der Ansprüche 8 bis 11, **dadurch gekennzeichnet, dass** die Evaluierungsvorrichtung die Höhe der Schale durch einen Vergleich des Versatzes des Lichts, das von dem Positionierungsbereich oder einem Bereich in der Nähe des Positionierungsbereichs reflektiert wird, mit dem lasergenerierten Lichtbereich auf der Schale, bestimmt.

13. Kontrollvorrichtung für Lebensmittel nach einem der vorangehenden Ansprüche, **dadurch gekennzeichnet, dass** die Evaluierungsvorrichtung einen Datenspeicher zum Speichern von Produkterkennungsdaten und Produktsortierungsdaten umfasst und einen Lernmodus zum Erfassen von Produkterkennungsdaten und Produktsortierungsdaten neuer Produkte aufweist.

14. Verwendung der Kontrollvorrichtung nach einem der Ansprüche 1 bis 13 zur Identifizierung und/oder Sortierung von in einer Schale verpackten Lebensmitteln.

## Claims

1. Food product checking apparatus for identification and/or grading of food products packed in a tray, comprising a positioning area, an illumination device for illuminating the food product with white light, at least one camera for taking images of the illuminated food product and an evaluation device for image processing, wherein the evaluation device performs an identification of the food products by color analysis and/or brightness analysis of the images taken by the camera, **characterized in that** the evaluation device identifies the contour or shape of the food product in the tray by a contour filter or an edge filter, thus distinguishing the shape of the food product from the tray and identifying inclusions in a seal by comparison with target data of packaging free from defects.

2. Food product checking apparatus in accordance with claim 1, **characterized in that** the illumination device comprises light sources for red, green and blue light, said light sources emitting light in a direction towards a reflection area where the emitted light is mixed into white light.

3. Food product checking apparatus in accordance with claim 2, **characterized in that** a light source has at least one light emitting diode, wherein the light emitting diode emits red or green or blue or white light and preferably wherein a polarisation filter is provided for polarising the emitted light.

4. Food product checking apparatus in accordance with any one of claims 1 to 3, **characterized in that** the positioning area comprises an enclosure having openings allowing a through-put of food products and/or trays and **in that** a conveyor belt is provided for transporting the food products and/or trays.

5. Food product checking apparatus in accordance with any one of claims 1 to 4, **characterized in that** color reference fields are arranged at or close to the positioning area within the vision range of at least a first camera, preferably wherein a color reference field is used for each of the colors red and/or green and/or blue and/or white.

6. Food product checking apparatus in accordance with any one of claims 1 to 5, **characterized in that** the evaluation device is adapted to identify fat areas and/or lean areas and/or bone areas in meat products or sausage products by analyzing an image with regard to the positions of the colors red, green and blue and preferably by analyzing the brightness thereof.

7. Food product checking apparatus in accordance with any one of claims 1 to 6, **characterized in that** the evaluation device identifies the shape and/or size of the food products in the tray and/or the shape and/or size of the trays and/or a shape distribution and/or the size of fat areas and/or lean areas and/or bone areas of the food product.

8. Food product checking apparatus in accordance with any one of claims 1 to 7, **characterized in that** it comprises a laser device (62) which produces at least one laser-generated light line across the tray, preferably by emitting green light at an angle relative to a plane perpendicular to the positioning area.

9. Food product checking apparatus in accordance with claim 8, **characterized in that** the evaluation device identifies laser-generated light lines in a taken image and analyzes said laser-generated light lines with regard to segmentation, particularly wherein the evaluation device determines the number of the segments and/or the length of the segments and/or the length distribution of the segments and/or the angle of the segments, and/or the angle distribution of the segments and/or the offset between the segments.

10. Food product checking apparatus in accordance with claim 8 or 9, **characterized in that** the evaluation device analyzes the texture of the food product in the area of a laser-generated light line.

11. Food product checking apparatus in accordance with any one of claims 8 to 10, **characterized in that** the evaluation device determines the dome height of a lid of the tray by comparing the reflection light line of a laser-generated light line achieved by light reflection on the sealed see-through lid with the light reflected on a tray edge.

12. Food product checking apparatus in accordance with any one of claims 8 to 11, **characterized in that** the evaluation device determines the height of the tray by comparing the offset of the light reflected from the positioning area or an area close to the positioning area with the laser-generated light area on the tray.

13. Food product checking apparatus in accordance with any one of the preceding claims, **characterized in that** the evaluation device comprises a data storage for storing product identification data and product grading data and comprises a learn mode for acquiring product identification data and product grading data for new products.

14. Use of the food product checking apparatus in accordance with any one of claims 1 to 13 for identification and/or grading of food products packed in a tray.

## Revendications

1. Dispositif de contrôle pour produits alimentaires en vue de l'identification et/ou du triage de produits alimentaires emballés dans une coque, comprenant une zone de positionnement, un dispositif d'éclairage pour l'éclairage du produit alimentaire avec une lumière blanche, au moins une caméra pour l'enregistrement d'images du produit alimentaire éclairé et un dispositif d'évaluation pour le traitement de l'image, dans lequel le dispositif d'évaluation effectue une reconnaissance des produits alimentaires au moyen d'une analyse des couleurs et/ou d'une analyse de la luminosité des images enregistrées par la caméra, **caractérisé en ce que** le dispositif d'évaluation reconnaît, au moyen d'un filtre de contour ou d'un filtre de côté, le contour ou la forme du produit alimentaire dans la coque et sépare ainsi la forme du produit alimentaire de la coque et reconnaît des inclusions dans un joint scellé par une comparaison avec des données théoriques d'un emballage sans défaut.

2. Dispositif de contrôle pour produits alimentaires selon la revendication 1, **caractérisé en ce que** le dispositif d'éclairage comprend des sources de lumière pour une lumière rouge, verte et bleue, qui émettent la lumière en direction d'une zone de réflexion mélangeant la lumière émise en une lumière blanche.

3. Dispositif de contrôle pour produits alimentaires selon la revendication 2, **caractérisé en ce qu'**une source de lumière présente au moins une diode électroluminescente, dans lequel la diode électroluminescente émet une lumière rouge ou verte ou bleue ou blanche et il est prévu au moins un filtre de polarisation pour la polarisation de la lumière émise.

4. Dispositif de contrôle pour produits alimentaires selon l'une quelconque des revendications 1 à 3, **caractérisé en ce que** la zone de positionnement présente une enceinte, qui est dotée d'ouvertures pour le passage de produits alimentaires et/ou de coques et il est prévu une bande transporteuse pour le transport des produits alimentaires et/ou des coques.

5. Dispositif de contrôle pour produits alimentaires selon l'une quelconque des revendications 1 à 4, **caractérisé en ce que** des champs de référence de couleurs sont disposés sur ou à proximité de la zone de positionnement, à l'intérieur d'un champ de vision d'au moins une première caméra, dans lequel on utilise de préférence un champ de référence de couleur pour chacune des couleurs rouge et/ou vert et/ou bleu et/ou blanc.

6. Dispositif de contrôle pour produits alimentaires selon l'une quelconque des revendications 1 à 5, **caractérisé en ce que** le dispositif d'évaluation est conçu de façon à reconnaître des zones grasses et/ou des zones maigres et/ou des zones osseuses dans des produits de boucherie ou de charcuterie, en analysant une image quant aux positions des couleurs rouge, vert et bleu et de préférence quant à leur luminosité.

7. Dispositif de contrôle pour produits alimentaires selon l'une quelconque des revendications 1 à 6, **caractérisé en ce que** le dispositif d'évaluation reconnaît la forme et/ou la grandeur des produits alimentaires dans la coque et/ou la forme et/ou la grandeur des coques et/ou une répartition de formes et/ou la grandeur de zones grasses et/ou de zones maigres et/ou de zones osseuses du produit alimentaire.

8. Dispositif de contrôle pour produits alimentaires selon l'une quelconque des revendications 1 à 7, **caractérisé en ce qu'**il comprend un appareil laser, qui produit au moins une ligne de lumière générée par laser vers la coque, en émettant de préférence une lumière verte sous un angle par rapport à un plan perpendiculaire à la zone de positionnement.

9. Dispositif de contrôle pour produits alimentaires selon la revendication 8, **caractérisé en ce que** le dispositif d'évaluation reconnaît des lignes de lumière générées par laser dans une image enregistrée et les analyse en ce qui concerne leur segmentation, dans lequel il détermine en particulier le nombre des segments, et/ou la longueur des segments, et/ou la répartition de longueur des segments, et/ou les angles des segments, et/ou la répartition des angles des segments et/ou le décalage entre les segments.

10. Dispositif de contrôle pour produits alimentaires selon la revendication 8 ou 9, **caractérisé en ce que** le dispositif d'évaluation analyse la texture du produit alimentaire dans la zone de la ligne de lumière générée par laser.

11. Dispositif de contrôle pour produits alimentaires selon l'une quelconque des revendications 8 à 10, **caractérisé en ce que** le dispositif d'évaluation détermine la hauteur de l'arrondi d'un couvercle de la coque par comparaison de la ligne de réflexion d'une ligne de lumière générée par laser, qui se forme par réflexion de la lumière sur le couvercle scellé transparent, avec la lumière qui est réfléchie sur un bord de la coque.

12. Dispositif de contrôle pour produits alimentaires selon l'une quelconque des revendications 8 à 11, **caractérisé en ce que** le dispositif d'évaluation détermine la hauteur de la coque par une comparaison du décalage de la lumière, qui est réfléchie par la zone de positionnement ou par une zone proche de la zone de positionnement, avec la zone de lumière générée par laser sur la coque.

13. Dispositif de contrôle pour produits alimentaires selon l'une quelconque des revendications précédentes, **caractérisé en ce que** le dispositif d'évaluation comprend une mémoire de données pour mémoriser des données de reconnaissance de produit et des données de triage de produit et présente un mode d'apprentissage pour saisir des données de reconnaissance de produit et des données de triage de produit de nouveaux produits.

14. Utilisation du dispositif de contrôle selon l'une quelconque des revendications 1 à 13 pour l'identification et/ou le triage de produits alimentaires emballés dans une coque.
